# EUROPEAN PATENT APPLICATION

(11) **EP 4 749 638 A1**
(43) Date of publication of application: **27.05.2026**
(21) Application number: 25217253.1
(22) Date of filing: 20.11.2025
(51) Int. Cl.: G16H 20/30, G16H 20/40, G16H 40/63

(54) **DATA STREAM RESPONSE REACTION IN A MULTI-SYSTEM INTERACTION**

(30) Priority: 20.11.2024 US 202418954216
(71) Applicant: Cilag GmbH International, 6300 Zug (CH)
(72) Inventor: HARRIS, Jason L., Cincinnati, 45242 (US); SHELTON IV, Frederick E., Cincinnati, 45242 (US); BRADY, John E., Cincinnati, 45242 (US); DECK, Andrew C., Cincinnati, 45242 (US); JONES, Shannon L., Cincinnati, 45242 (US); ARONHALT, Jacqueline Corrigan, Cincinnati, 45242 (US)
(74) Representative: Carpmaels & Ransford LLP

(57) **Abstract**

A surgical system may include a processor configured to obtain an input control data stream associated with a measurement. The input control data stream may be associated with a control loop of the surgical system. The processor may be further configured to determine an importance factor of a condition associated with a patient. The processor may be further configured to generate a response reaction based on the input control data stream and the importance factor of the condition associated with the patient. The processor may be further configured to determine a reaction time, the reaction time being a time period between an instant at which the input control data stream is obtained and an instant at which the response reaction is generated. The processor may be further configured to modify the response reaction based on the determined reaction time.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November20, 2024, entitled METHOD FOR MULTI-SYSTEM INTERACTION, and
- U.S. Patent Application No. 18/954,206, filed November 20, 2024, entitled DATA STREAMS MULTI-SYSTEM INTERACTION.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Systems, methods, and instrumentalities are disclosed herein for a (e.g., pre-, in-, and/or postoperative) patient monitoring system. A surgical system may include a processor. A surgical system may include a processor configured to obtain an input control data stream associated with a measurement. The input control data stream may be associated with a control loop of the surgical system. The processor may be further configured to determine an importance factor of a condition associated with a patient. The processor may be further configured to generate a response reaction based on the input control data stream and the importance factor of the condition associated with the patient. The processor may be further configured to determine a reaction time, the reaction time being a time period between an instant at which the input control data stream is obtained and an instant at which the response reaction is generated. The processor may be further configured to modify the response reaction based on the determined reaction time.

The modification of the response reaction may be an escalation. The modification of the response reaction may be a recession. The reaction time may be based on the importance factor of the condition associated with the patient. The importance factor may be based on a patient risk. The instant the input control data stream is determined when the input control data stream may violate a first threshold associated with the input control data stream. The instant the input control data stream is determined when the input control data stream may satisfy a second threshold associated with the input control data stream.

The control loop of the surgical system may be a closed loop system. The closed loop system of the surgical system may be changed to an open loop system. The processor may be further configured to prevent an anticipated instability from affecting the surgical system based on a change in the first data stream or the second data stream. The surgical system may be a heating system of a patient. The measurement associated with the input control data stream may be a temperature of the patient. The condition associated with the patient may be a risk of overheating and the importance factor may be high. The response reaction may be generated to reduce the temperature of the patient based on the input control data stream and the importance factor of the condition associated with the patient. The reaction time may be the time period between an instant at which the input control data stream is obtained and an instant at which the response reaction is generated. The response reaction may be modified based on the determined reaction time.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIG. 6 shows an example computer-implemented surgical system for determining a control signal.
FIG. 7 shows an example computer-implemented surgical system for determining a body temperature management control signal.
FIG. 8 shows an example flowchart for determining a control signal.

### DETAILED DESCRIPTION

A more detailed understanding may be had from the following description, given by way of example in conjunction with the accompanying drawings.

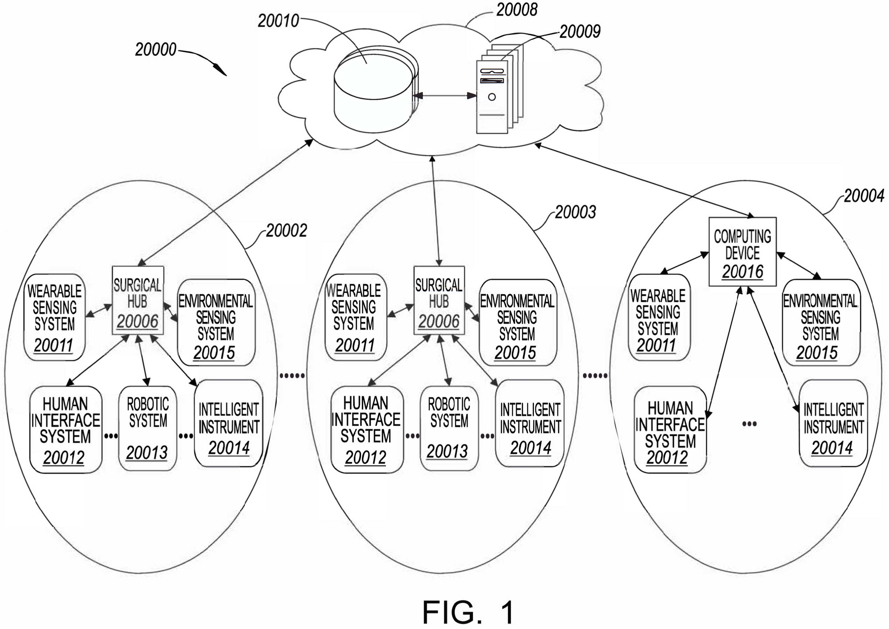

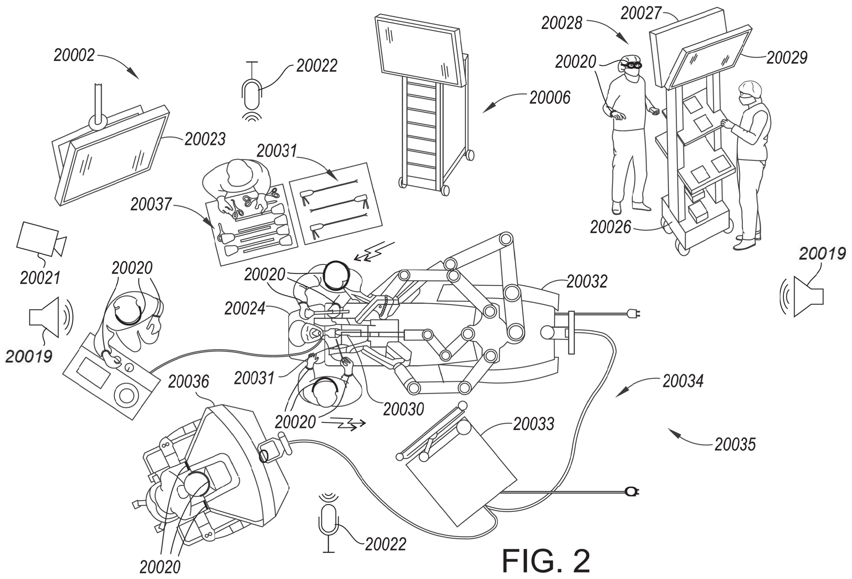

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

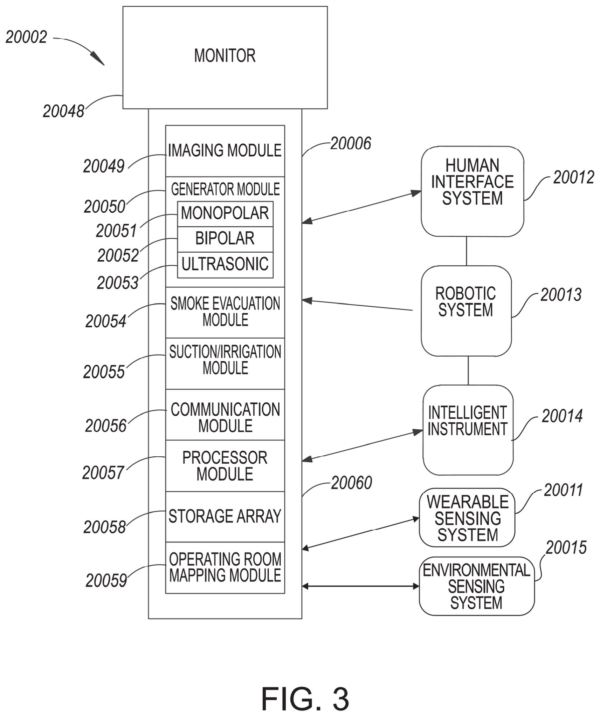

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

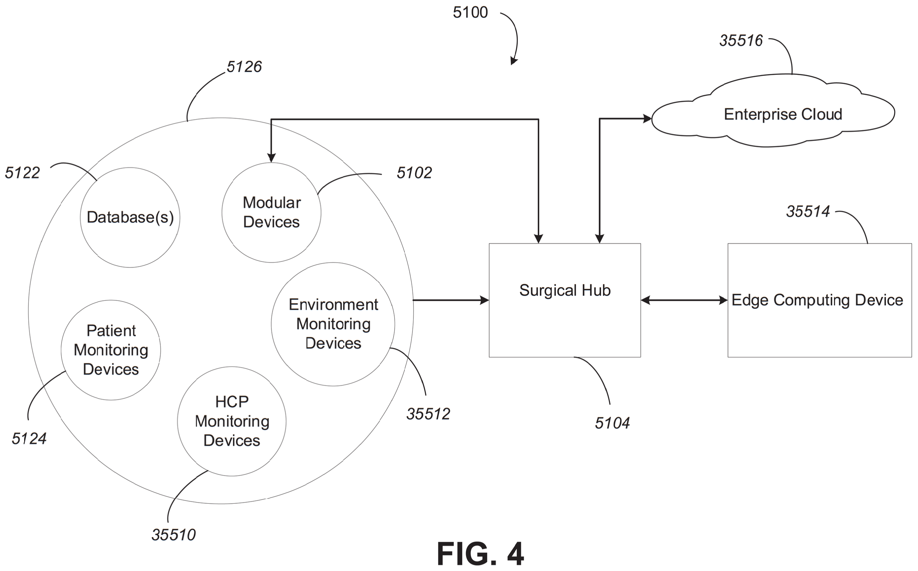

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an **RF** electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

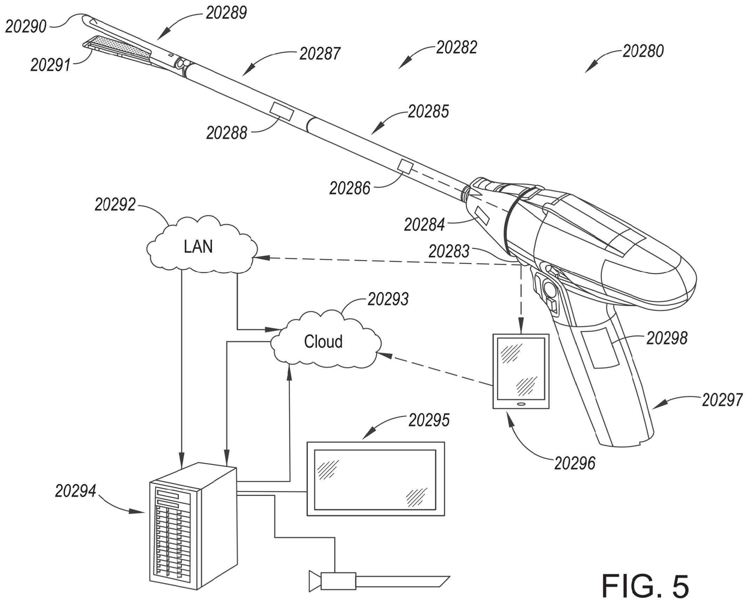

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

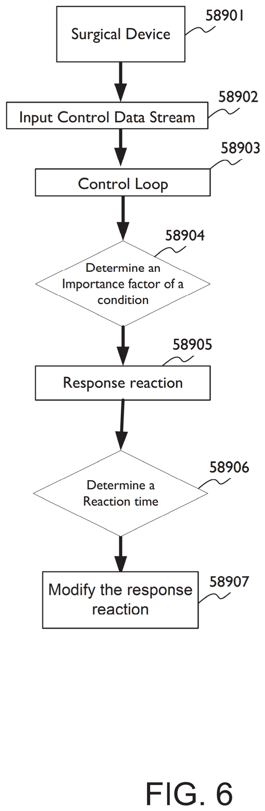

FIG. 6 shows an example computer-implemented surgical system for determining a response reaction and modifying the response reaction. The surgical system may include or may interact with a surgical device 58901. The surgical device 58901 may output an input control data stream 58902. The input control signal may be associated with a control loop 58903. An importance factor of a condition may be determined at 58904. The importance factor may refer to a numerical value indicative of at least one of a risk level, an urgency, and a priority assigned to a condition affecting a patient; the importance factor may be based on one or more criteria, for example at least one of patient risk, severity, and likelihood of complications. At 58905, a response reaction may be determined. A reaction time may be determined at 58906. The response reaction may be modified at 58907.

The system may not respond to a signal change. If the system is unable to decide what to do, the system may set an alarm condition. The presence of a surgeon may be dependent on the alarm system. The system may actively identify if the surgeon is present. These methods to detect surgeon presence include but are not limited to an eye tracking and/or camera system. If the system requires a response, it may generate an alarm condition.

Alarms for a healthcare professional (HCP) may go beyond the presence of a surgeon. The alarms may call for a scrub tech, an anesthesiologist, etc. Event timestamping and timeliness watchdogs may be provided. Data may be time stamped to understand the timeliness associated with it, and corresponding watchdogs may be implemented. If a watchdog service expires (or is not satisfied), then the system may escalate to a potential action.

Potential actions may be taken without surgeon feedback. Reversion to an immediately prior value may be the action selected, including but not limited to, reversion to a default state, change to a non-clinical state, proceeding with a 'best' action, proceeding with a 'safest' action, etc. Predefined actions for when a timer expires may be provided. Prior to the surgery occurring, the surgeon or HCP may define what will happen in the event that the surgeon or other HCP is not able to provide feedback in a sufficient amount of time.

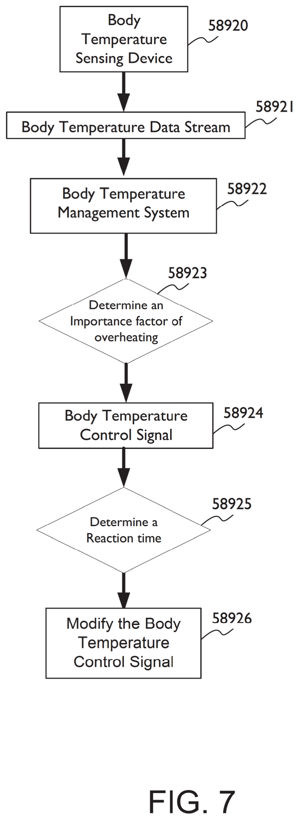

FIG. 7 shows an example computer-implemented surgical system for determining a response reaction in a body temperature control signal 58924 and modifying the body temperature control signal 58924 based on a determined reaction time 58925. A body temperature sensing device 58920 may detect a body temperature of a patient and may output a body temperature data stream 58921 which represents the body temperature of the patient. The body temperature of the patient represented as a data stream may be fed to a body temperature management system 58922. The body temperature control signal may be modified 58926.

The surgical device 58901 may include, but not be limited to, temperature monitors (e.g., thermometers), pulse oximeters, blood pressure monitors, capnographs, electrocardiograms, spirometers, intraoperative blood glucose monitors, intraoperative fluid monitors, intraoperative nerve monitors, hemoglobin monitors, intraoperative pressure monitors, electroencephalography (EEG) monitors, ultrasounds, electromyography (EMG) devices, dosimeters, or non-invasive cardiac output (NICOM) monitors. The surgical device 58901 may output a data stream (e.g., an input control data stream 58902, or a body temperature data stream 58921) associated with a measurement, such as a measurement of the patient (e.g., a temperature, or O₂ level) and a control loop 58903 (e.g., increasing or decreasing a patient temperature using a temperature management system 58922).

The temperature data stream, for example, may be obtained via a body temperature measuring device 58920 (e.g., an esophageal temperature probe, nasopharyngeal probe, skin surface thermometer, infrared or tympanic membrane thermometer, etc.). The body temperature data stream may be sent to a temperature management system 58922 that may change the body temperature of the patient (e.g., using forced-air warming systems like a Bair Hugger^{™}, a fluid warming system using intravenous (IV) fluids, blood, or other fluids administered to the patient during surgery, a heated mattress or pads like a HotDog^{™} patient warming system, water-circulating warming/cooling systems like a Cincinnati Sub-Zero (CSZ) Blanketrol^{™}, intravascular temperature management systems, warming blankets, radiant warming devices, heat and moisture exchange (HME) filters, warming lights, etc.).

Maintaining a stable, comfortable temperature for the patient may be difficult without the temperature monitoring system and the ventilator being able to communicate. A delay in the feedback control system (e.g., a delay in the cold air being sent to the patient and a measured body temperature of the patient) may have negative effects on the response of the system. The response of the system may include offsetting the control action, oscillations, instability, and/or performance degradation.

The modification of the response reaction may be an escalation. For example, the response reaction as an escalation may be the body temperature management system indicating to raise the body temperature of the patient. The modification of the response reaction may be a recession. For example, the response reaction as an escalation may be the body temperature management system indicating to lower the body temperature of the patient.

The reaction time may be determined based on the importance factor of the condition associated with the patient, for example, a risk of the patient overheating may be a condition with a high importance. For example, an importance factor associated with overheating may be determined 58923. The importance factor may be based on a patient risk, so for a patient more prone to overheating due to age, previous medical conditions, or the like, the importance factor may be determined to be higher, for example, than a patient who is younger, has no previous medical conditions, etc.

An instant of the input control data stream is determined may be where the input control data stream violates a first threshold associated with the input control data stream, for example, when a body temperature of a patient raises above the critical temperature level determined to cause the patient to overheat. In other words, the instant may correspond to the point in time at which the input control data stream violates (e.g. exceeds) the first threshold. An instant the input control data stream is determined may be where the input control data stream satisfies a second threshold associated with the input control data stream. In other words, the instant may correspond to the point in time at which the input control data stream satisfies the second threshold.

The control loop of the surgical system may be a closed loop system or may be changed from an originally closed loop system to an open loop system. An anticipated instability may be prevented from affecting the surgical system based on a change in the first data stream or the second data stream, for example, if a body temperature of a patient is determined to be rising, preemptive action may be taken by the body temperature management system to prevent a case of overheating for the patient. An anticipated stability may correspond to a predicted physiological deviation from a predefined range or threshold.

Primary surgical actions and secondary surgical actions may be provided. A primary surgical action may be defined as a critical surgical step, such as one performed by the surgeon themselves. A secondary surgical action may be one that is supporting primary surgical steps but performed by someone else. This could be control of anesthesia, preparation of devices, etc. In examples, a surgeon may be sitting at the console performing an action, and an arm has been retracted to perform a reload. The reload action still may not be performed within several minutes. In examples, patient temperature may be dropping, and O2 supplementation may slow try to accommodate this. Then the temperature system may drop. The system may hold the level and/or pause until the surgeon is available. Required response times from a surgeon may be variable depending on the type of surgical action. Categorization of these actions may then influence how the system informs and/or responds to the scenario.

Timeliness of response predicted on the severity of the action may be provided. In examples, a scenario in which a critical structure may have been inadvertently damaged may require a much faster HCP response than a scenario where the risk is significantly lower.

A timing of a response based on the response strategy may be provided. Prior decisions and/or surgical goals may have been previously categorized as reactive, proactive, preemptive, predictive, preventative, etc. How the system has categorized these responses may then inform the next steps and timing for the responses, knowing that the overall category it was working within was previously defined.

Minimum required signal change for system action may be provided. Not all stimuli may result in a signal change. Small signals may be filtered out and/or prevent the system from over-compensating to what may be overall small changes within the procedure. The system may identify the signal change, but mitigation may not be activated.

A signal alarm may be provided. If the system fails to respond to a signal change, and the system may internally detect the lack of response, the system may create a signal alarm. The system may fail to identify the signal change. Secondary gating control for the system may be provided. The system may correlate certain procedure steps or segments to other secondary control inputs. These may be a sequence of operations, heartbeat data from equipment, or other controls to ensure the system is operating within compliance. In examples, the surgeon may move to the next surgical step, but there may be no change in any of the system inputs. This may indicate that there may be an error in equipment monitoring.

Timer alarms may be provided. Timer alarms may be preemptive, predictive, preventative, etc. An absence of a change in state may be provided. The system would have expected (based on a secondary, controlling or gating input) that a step should have occurred. Since the step should have occurred, and has not yet, the system should take a pre-emptive step to control for that. In examples, O2 may drop and a bear hugger may be turned on. In examples, in the context of an AFIB and a patient with hypothermia, the system may reduce O2 supplementation to preemptively adjust for metabolic slowing. In examples, moving a stapler to clamp on a stomach, an arm with an endocutter may be approaching a collision/minimum distance with arm holding scope outside of the patient. In examples, a first arm may be within a certain distance of a second arm. The system may pre-emptively move the second arm out of the way so that the first arm does not collide.

A simulation may be provided. In examples, in a pre-procedure trocar placement simulation context, simulation may be used to evaluate robot arm movements required to complete the procedure and provide recommendation for ideal trocar position to avoid collision. Simulation may further be provided to inform which tools to place in each arm to enable access and avoid collision. A surgeon may move a first arm to avoid joints externally and an internal tool of the second arm. A simulation may be run to inform ideal position with maximum available displacement.

An OR space may be moved via a third input (e.g., move the stomach to a more ideal location using arm C with a grasper tool). The system may predict these behaviors outside of using the procedure plan. In examples, a robot arm with an 'efficiency' reset may be provided. A complicated procedure step may require extra motion/extension. The robot may continue with the arm fully extended and/or it may preemptively retract the joint to a maximum 'efficiency' placement. In examples, data may be fed over time, and a data feed may drift out of range. The system may preemptively restart and/or reset to reduce error. In examples, a computer may restart mid-day. In examples, data reduction may be escalated from 2 to 3 data sources, and at some point the system may reduce the number of streams to avoid overloading the data pipe.

Predefined thresholds of behaviors to determine legitimacy may be provided. The system may monitor the overall status of signals, and/or actions, relative to predefined thresholds. When the signal starts to move outside of those thresholds, it may become an indication that the signal may warrant a pre-emptive response. These thresholds or guard bands (e.g., control points) may not necessarily be the same as critical fault failures but may provide early indications for the system to take action.

Monitoring of the variability of the signal may be provided. Signals may be monitored not just to their absolute value, but to the amount of variability present within their data stream. If the variability increases over time, it may be an indication of equipment failure or other problems within the system that need to be addressed. Signal standard distribution and associated statistical variability may be monitored. Intra-data stream variability, such as point-point variability with distinct datapoints may be provided.

System health monitoring of a characterized system may be provided. A biological passport (e.g., a corollary) and it's use with detecting doping in professional sports may be provided. A characterized system may have a unique and/or distinct number of characteristics to it. A unique profile may be created relative to the characteristics of the specific equipment. Examples of these characteristics may include the amount of force to move certain joints for nominal movements, power consumption in standby, or other indications that fall within the normal operational conditions but are unique to this system. The system may monitor for changes in these unique parameters as a method to predict when an error is occurring within the system, or performance may be slowly degrading over time.

This response may occur for an imbalanced number of unknowns. If an additional unknown is predicted, this response would add an equation before the system is imbalanced. In examples, monopolar application may cause an interference with the EKG, transcutaneous, and other patient attached monitors and may be ignored or put into open loop condition as an anticipation of the upcoming occurrence and the prevention of the occurrence from causing the unbalance or instability all together.

Importance may be high as the interference from the monopolar device may cause false alarms on the EKG for heart stoppage, may interfere with pacemaker controls, even may interfere with closed loop ventilator controls. The decisiveness is also of high important as the corrective action needs to be switch to before any of the critical risks to patient health monitor are triggered.

Preventing an anticipated instability from changing the controlled system may be based on a change from the input system (e.g., prevents a change). A proactive response to an external data stream trigger that may affect the operation or stability of a closed loop feedback system due to a potential input instability may be provided. Triggers for detectable events/trends that may generate an anticipatory trigger may be provided.

A rate of change of a signal may preempt a threshold crossing scenario where the derivative (rate of change) of a parameter may indicate a fault or error that may occur prior to an actual threshold boundary being crossed. In examples, a powered endocutter may encounter a stall condition when the force encountered by the drivebar exceeds the capacity of the motor gearbox system to provide sufficient force to continue moving forward. When a true stall is encountered, removal of the endocutter may become more difficult. This stall condition may be detected as well by monitoring the force being current generated in relation to other parameters. During a drive action, the rate of change of force may instead be monitored, and if force is accelerating rapidly, may predict when a stall condition may occur. This may allow the stall condition to potentially be prevented from occurring in the first place, and alert the system that the tissue and/or firing location may warrant further inspection.

Impacts of differing external signal variation may be provided. The signal that the closed loop system is tracking may vary irregularly in both directions from zero. A measurement context to intelligently interpret the signal may be provided. Viable changes, limits and physiology behavior may be used to identify what portions of the signal may be real. O2 requirements for the body for instance may not need second by second gross adjustment. In the case of a noisy signal, the input feed may be smoothed or averaged over a time frame like 1 minute which may remove the instability of the closed loop but not require it to seek another input source. Pacemakers and EKG leads, for example, don't have the freedom to average overtime without effecting the primary short-term function of the closed loop system. This example, in contrast, may resolve the input signal irregularities directly rather than using time to average out the overlaid error.

Intermittent signal loss may occur. A signal may remain somewhat stable when active but may have dropouts that induce instability on the closed loop system if every data point is reacted to. Time dependent averaging may be used to smooth the signal, depending on what percentage of the time is drop out. The challenge would be drifting or varying real adaptations could be masked by the drop out timing and the smoothing.

Characterization of the signal loss may be provided. If the loss portion may be easily identified (i.e. zero, +12V or -12V) then these data points may be filtered out and removed leaving just real signal changes and the transition zones. The signal may be smoothed or cleaned with other transformations to lose less data.

In the scenario where the signal is trending toward a threshold, but the threshold has not yet been reached/exceeded, a change in the data stream (trend/slope/rate) may occur. The number of times a triggering event has impacted closed loop behavior in the recent past may be provided. In examples, a moving stapler may clamp on the stomach, an arm with an endocutter may approach a collision/minimum distance with the arm holding the scope outside of the patient. The system may notify/warn surgeon that arm is approaching the threshold. The first arm may be within a certain distance of the second arm. The data frame rate may be increased for resolution of the arm position to better improve the ability of system to avoid collision. The first arm may be within a certain distance of the second arm. The slow speed of the arm movement may prevent over-accelerating the first arm forward into the second arm.

Responses to control signal instabilities may be provided. An upcoming procedural step may impact the datastream. Catching a trigger may help minimize overshoot. An alternative system data feed may be utilized as a means to anticipate the initiation of the interdependent system. In examples, Hugo Robot may be able to solve position of tools within the patient and may know the position of each tower. In order to prevent collision/entanglement the robot may select optimal joint movement to achieve procedural steps. To do so, it may solve for position of the external portion of the arms, which require additional data input via simulation, OR cameras, etc. In examples, in an AFIB context, a patient temperature may begin to drop but CO2 outgassing may be constant. The system may reduce O2 supplementation to preemptively adjust for anticipated reduction of metabolism.

Circumstances when predictive behavior is beneficial may be provided. Physiologic patient changes may make predictive behavior beneficial. In examples, a body is a closed loop system outside of the system closed loop control. This level may allow the system closed loop to adjust as patient parameters shift. Minor changes in the operating procedure may be provided. The surgeon may deviate slightly from a procedure plan, which means the system may accommodate the changes. Major changes in a planned procedure may be provided. Complications or significant deviations may occur during the surgery, and as a result cause a significant change from how the surgery was originally planned. In examples, during a laparoscopic procedure, a surgical error may occur which leads to the surgeon converting the surgery to an open procedure. As the decision is made to convert the procedure to open, the system may move pieces of equipment out of the way, start to disengage pieces of equipment or ensure tools are ready for extraction.

In other situations, equipment failures may occur. A failure in equipment may occur during the procedure, which may cause the system to react to that failure. A current surgeon may be substituted during the procedure. Within a residency program or training program, an attending surgeon and resident/trainee surgeon may alternate portions of the surgery. In examples, a highly skilled surgeon may move through surgery in a much more confident and overall faster pace than a resident or novice surgeon. As a result, the system may anticipate when they are ready to move to the next step. A more novice surgeon may take longer during a surgery and may be more likely to pause or contemplate next moves. As a result, the system may want to be less reactive to avoid presenting the opportunity for confusion.

In examples, 2 closed loop systems may not be dependent from each other, but may impact each other's function. In a predictive kinematic simulation, a patient or robot movement may be optimized for movement efficiency purposes. Simulation of intermediary limbs and potential collisions may be provided. A simulation initiation driven by the system detection of a joint or trunk prior to the end effector may prevent the end effector from reaching its desired location. If preventatively something is determined to be going wrong, a simulation and/or forecast may be used to predict how and/or why. In examples, a robot arm position may require preventative and/or tactical decision for arms. The system may define which positions are/ are not satisfactory or are most likely to be satisfactory.

A single quadrant procedure and a multi quadrant procedure may be provided. A robot may move tools to different locations where ideal set up differs. The system may move arms before this. An image of tangled arms vs not tangled arms may help determine if heading in the wrong direction. In comparing a planned simulation to an updated simulation, an initial plan may include actual procedure steps and if the surgeon doesn't follow plan, it may be corrected. Context of the data for the specific treatment may be based on globally accessible data. Context relative to an encountered irregularity may include radiology, histology, etc. Data available to enable a predictive simulation may include patient specific data like medication data, allergies, a non-allergic response to prior medications, current medications and dosages, etc. A response to prior procedures may include a procedure location/known adhesions. Doctor specific responses may include where is the simulation displayed, the preferred language, the preferred simulation display mode, where within the hospital, and device data may include EES owned data, Non-EES owned data, a smart device, non-smart device, historical data, etc. Data categories may be used to perform assessment of the patient, environment, surgeon, etc.

Predictive forecasting may use the data streams at hand and metadata or context to predict the current trend causing a trigger in the future and therefore some action in the present may be necessary. Anticipation of an instability or change based on the input stream that is undesirable and critical to function (prepares for a change) may be provided. Adaption may be a reaction. A monitored parameter within or outside of the system may have single or double end bounded thresholds. Exceeding these thresholds may provoke a response.

In examples, a smoke evacuator activation of an energy device may be provided. The motor on the smoke evacuator may be activated. In example, for an insufflation pump, reduction of the intraperitoneal insufflation pressure may be below a predefined range 5-7mmHg (Low), with a normal operating range of 12-15mmHg. In examples, for an endocutter, a motor current may be above an acceptable range within a mechanical lockout zone (e.g., >60lb within the first 0.250" of I-beam / knife travel).

In examples, for a bipolar generator, adjustment of the operational voltage level once the tissue impendence may exceed 150ohms. A bipolar system may run in a constant current mode until it may be run in constant power mode and then in constant voltage mode. This may be due to the tissue impendence and the high levels of current needed when the impendence is low. This may be a response to the generator not the tissue. The generators may not be capable of outputting the power needed in low impedance portions of the tissue impendence curve to run in the more controlled settings.

In examples, for a powered endocutter, powered stapling may use a threshold of motor current or drive bar speed relative to the control system indicated speed as a means to control loading on the end-effector. In the case of a motor current monitored system the current may be a direct reflection of the force / torque the moor is having to overcome. There may be a threshold on the max current level which may be used to trigger a reduction in motor speed which may result in a related reduction since the tissue in the jaws is viscoelastic. There may be a condition with this manner on control on the motor current which may be detrimental if the speed is reduced. The rotor lock condition, which may occur when the motor ceases to spin, may result in the motor current going to its maximum level. A reduction in voltage or current may not change whether the motor was able to spin, and the continued current may heat up the motor causing further reduction in output torque relative to input current. In this case, the motor may need to be shut off entirely, not just slowed down. To determine this, the data feed needed may be the encoder on the motor or the drive rack which measure the advancement rate of the system rather than the current through the motor. This relative adaptation may be triggered based on a secondary monitor of speed and below a certain threshold speed rather than current could be monitored for the critical shut down trigger. The motor current may be used up to a pre-specified max at which point the control input stream would need to be moved from current to speed.

Importance may be low (motor over heating is not a risk to the patient just the device and a delay of the procedure) so timeliness of a fast fuse trigger that is reset able would be fine. It may not cause a cascade of other detrimental issues to the need for a decisive answer is also not high.

Monitored parameters may detect an unacceptable condition in an unexpected portion of the operational step. In examples, for a bipolar generator, low measured bipolar electrode impendence (0-5 ohms) may indicate either immersion in fluid, low impendence tissue, or an electrical short.

A reactive condition may be eliminated, for example, by switching a data source. Short term change in the closed loop-controlled system based on a violation of a threshold monitoring on the input stream may be a triggering event for response. Changes in the data that may impact the control loop (immediate results) include, but are not limited to, crossing a threshold (reactive), undesired patient status/critical event, rate of approaching a threshold, etc. The reaction may be immediate (quick fuse) or may be delayed (slow or delayed fuse) to determine if the response only momentarily violated the threshold and then returned to acceptable levels. The rate may be the rate at which a measure is approaching the trigger threshold. The rate may be the number of times it has approached the threshold. The rate may be based on how close the stream is to the threshold and the relative stability of the measure compared to the volatility of the measure. A related physiologic measure may approach a tipping point on the valid of the control stream so the system changes the data stream it is controlling off of before a threshold is ever exceeded (preventive). In an example, the core body temperature may approach a level that is likely to induce vasoconstriction or vasodilation which may result in an increase or decrease in blood volume per beat which may result in a change in heart rate which is what is being timed for the in-between beat application of energy for destroying the nerves resulting in the irregular heartbeat.

Data shifts may be monitored. Previous trigging events may be translated in larger or smaller results than expected (repetition or time based results). Initial fault response may not correct and/or resolve a data stream. In examples, a patient blood pressure may be elevated, max medication may be given, but a patient may not respond to the dose.

In examples, patient temperature may be low, a bear hugger may be turned on (and functioning properly) but the patient temperature may not increase. The anticipated behavior may not match data stream reaction to the action that was taken. The system and/or device may not function as intended. In examples, energy activation on patient heart, do not see increase in temperature on local cooling system as expected. Change may be anticipated but not shown/experienced in data.

A system and/or device may be operating as intended but may not meet the needs of the patient and/or surgeon. In examples, a local heart may cool on the highest setting and core. In examples, a harmonic blade may squeal. The user experience may be negative but function correctly. Data interaction may not meet expectations. Procedure plan may deviate (implications on future results). A surgeon may behave differently than intended. A device may not match the procedure plan sequence. The system may need to determine if these deviations are acceptable or harmful to control loop.

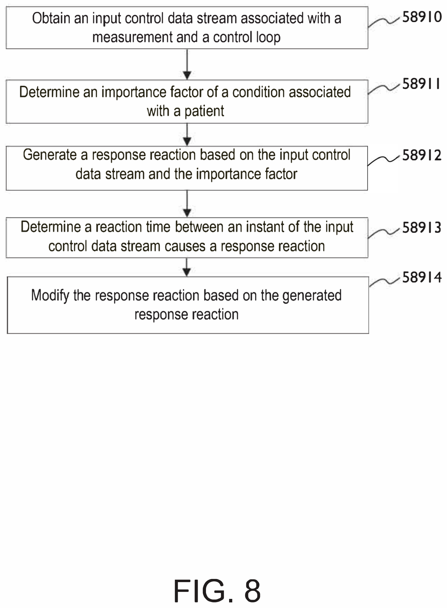

FIG. 8 shows an example flowchart for determining a response reaction and modifying the response reaction. At 58910, an input control data stream associated with a measurement (e.g. a measurement of or associated with a patient) and control loop may be obtained. At 58911, an importance factor of a condition associated with a patient may be determined. At 58912, a response reaction based on the input control data stream and the importance factor may be generated. At 58913, a reaction time may be determined, the reaction time being a time period between an instant at which the input control data stream is obtained and an instant at which the response reaction is generated. At 58914, the response reaction may be modified based on the reaction time.

In examples, an input control data stream associated with a measurement (e.g. a measurement of or associated with a patient) may be obtained. The input control data stream may be associated with a control loop. An importance factor of a condition associated with a patient may be determined.

Example 1. A surgical system comprising:
a processor configured to:
obtain an input control data stream associated with a measurement, wherein the input control data stream is associated with a control loop of the surgical system;
determine an importance factor of a condition associated with a patient;
generate a response reaction based on the input control data stream and the importance factor of the condition associated with the patient;
determine a reaction time between an instant of the input control data stream causes a response reaction to be generated; and
modify the response reaction based on the generated response reaction.

Example 2. The surgical system of example 1, wherein the modification of the response reaction is an escalation.

Example 3. The surgical system of any of examples 1-2, wherein the modification of the response reaction is a recession.

Example 4. The surgical system of any of examples 1-3, wherein the reaction time is based on the importance factor of the condition associated with the patient, and wherein the importance factor is based on a patient risk.

Example 5. The surgical system of example 4, wherein the instant the input control data stream is determined when the input control data stream violates a first threshold associated with the input control data stream.

Example 6. The surgical system of any of examples 4-5, wherein the instant the input control data stream is determined when the input control data stream satisfies a second threshold associated with the input control data stream.

Example 7. The surgical system of any of examples 1-6, wherein the control loop of the surgical system is a closed loop system.

Example 8. The surgical system of example 7, wherein the closed loop system of the surgical system is changed to an open loop system.

Example 9. The surgical system of any of examples 1-8, wherein the processor is further configured to: prevent an anticipated instability from affecting the surgical system based on a change in the first data stream or the second data stream.

Example 10. The surgical system of any of examples 1-9, wherein:
the surgical system is a heating system of a patient;
the measurement associated with the input control data stream is a temperature of the patient;
the condition associated with the patient is a risk of overheating and the importance factor is high;
the response reaction is generated to reduce the temperature of the patient based on the input control data stream and the importance factor of the condition associated with the patient;
the reaction time determined is the time between an instant of the input control data stream and the instant a response reaction is generated; and
the response reaction is modified based on the generated response reaction.

Example 11. A surgical operating method comprising:
obtaining an input control data stream associated with a measurement, wherein the input control data stream is associated with a control loop of the surgical system;
determining an importance factor of a condition associated with a patient;
generating a response reaction based on the input control data stream and the importance factor of the condition associated with the patient;
determining a reaction time between an instant of the input control data stream causes a response reaction to be generated; and
modifying the response reaction based on the generated response reaction.

Example 12. The surgical operating method of example 11, wherein the modification of the response reaction is an escalation.

Example 13. The surgical operating method of any of examples 11-13, wherein the modification of the response reaction is a recession.

Example 14. The surgical operating method of any of examples 11-14, wherein the reaction time is based on the importance factor of the condition associated with the patient, and wherein the importance factor is based on a patient risk.

Example 15. The surgical operating method of example 14, wherein the instant the input control data stream is determined when the input control data stream violates a first threshold associated with the input control data stream.

Example 16. The surgical operating method of any of examples 14-15, wherein the instant the input control data stream is determined when the input control data stream satisfies a second threshold associated with the input control data stream.

Example 17. The surgical operating method of any of examples 11-6, wherein the control loop of the surgical system is a closed loop system.

Example 18. The surgical operating method of example 17, wherein the closed loop system of the surgical system is changed to an open loop system.

Example 19. The surgical operating method of any of examples 11-18, further comprising:

preventing an anticipated instability from affecting the surgical system based on a change in the first data stream or the second data stream.

Example 20. The surgical operating method of any of examples 11-19, wherein:
the surgical system is a heating system of a patient;
the measurement associated with the input control data stream is a temperature of the patient;
the condition associated with the patient is a risk of overheating and the importance factor is high;
the response reaction is generated to reduce the temperature of the patient based on the input control data stream and the importance factor of the condition associated with the patient;
the reaction time determined is the time between an instant of the input control data stream and the instant a response reaction is generated; and
the response reaction is modified based on the generated response reaction.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A surgical system comprising a processor configured to:
obtain an input control data stream associated with a measurement, wherein the input control data stream is associated with a control loop of the surgical system;
determine an importance factor of a condition associated with a patient;
generate a response reaction based on the input control data stream and the importance factor of the condition associated with the patient;
determine a reaction time, wherein the reaction time is a time period between an instant at which the input control data stream is obtained and an instant at which the response reaction is generated; and
modify the response reaction based on the determined reaction time.

2. The surgical system of claim 1, wherein the modification of the response reaction is an escalation or a recession.

3. The surgical system of claim 1 or claim 2, wherein the reaction time is determined based on the importance factor of the condition associated with the patient, and optionally wherein the importance factor is based on a patient risk.

4. The surgical system of claim 3, wherein the instant the input control data stream is determined when the input control data stream violates a first threshold associated with the input control data stream, and/or wherein the instant the input control data stream is determined when the input control data stream satisfies a second threshold associated with the input control data stream.

5. The surgical system of any one of the preceding claims, wherein the control loop of the surgical system is a closed loop system, optionally wherein the closed loop system of the surgical system is changed to an open loop system.

6. The surgical system of claim 1, wherein the processor is further configured to:
prevent an anticipated instability from affecting the surgical system based on a change in the first data stream or the second data stream.

7. The surgical system of claim 1, wherein:
the surgical system is a heating system of a patient;
the measurement associated with the input control data stream is a temperature of the patient;
the condition associated with the patient is a risk of overheating and the importance factor is high; and
the response reaction is generated to reduce the temperature of the patient based on the input control data stream and the importance factor of the condition associated with the patient.

8. A method performed by a processor of a surgical system, the method comprising:
obtaining an input control data stream associated with a measurement, wherein the input control data stream is associated with a control loop of the surgical system;
determining an importance factor of a condition associated with a patient;
generating a response reaction based on the input control data stream and the importance factor of the condition associated with the patient;
determining a reaction time between an instant of the input control data stream causes a response reaction to be generated; and
modifying the response reaction based on the determined reaction time.

9. The method of claim 8, wherein the modification of the response reaction is an escalation or a recession.

10. The method of claim 8 or claim 9, wherein the reaction time is determined based on the importance factor of the condition associated with the patient, and optionally wherein the importance factor is based on a patient risk.

11. The method of any one of claims 8 to 10, wherein the instant the input control data stream is determined when the input control data stream violates a first threshold associated with the input control data stream, and/or wherein the instant the input control data stream is determined when the input control data stream satisfies a second threshold associated with the input control data stream.

12. The method of any one of claims 8 to 11, wherein the control loop of the surgical system is a closed loop system, optionally wherein the closed loop system of the surgical system is changed to an open loop system.

13. The method of any one of claims 8 to 12, further comprising:
preventing an anticipated instability from affecting the surgical system based on a change in the first data stream or the second data stream.

14. The method of claim any one of claims 8 to 13, wherein:
the surgical system is a heating system of a patient;
the measurement associated with the input control data stream is a temperature of the patient;
the condition associated with the patient is a risk of overheating and the importance factor is high; and
the response reaction is generated to reduce the temperature of the patient based on the input control data stream and the importance factor of the condition associated with the patient.

15. A computer-readable medium comprising instructions which, when executed by a processor, cause the processor to carry out the method of any one of claims 8 to 14.
